(19) Europäisches Patentamt
European Patent Office
Office européen des brevets

(11) **EP 4 663 137 A1**

(12) **EUROPEAN PATENT APPLICATION**
published in accordance with Art. 153(4) EPC

(43) Date of publication:
**17.12.2025 Bulletin 2025/51**

(21) Application number: **24766978.1**

(22) Date of filing: **28.02.2024**

(51) International Patent Classification (IPC):
**A61B 17/16** *(2006.01)*    **A61B 17/56** *(2006.01)*

(52) Cooperative Patent Classification (CPC):
**A61B 17/16; A61B 17/56**

(86) International application number:
**PCT/JP2024/007225**

(87) International publication number:
**WO 2024/185607 (12.09.2024 Gazette 2024/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **03.03.2023 JP 2023033144**
**13.10.2023 JP 2023177827**

(71) Applicant: **Mochida Pharmaceutical Co., Ltd.**
**Tokyo 160-8515 (JP)**

(72) Inventors:
• **MIZUNO Hitoshi**
**Tokyo 160-8515 (JP)**
• **KUBOKI Takahiro**
**Tokyo 160-8515 (JP)**
• **NAGASE Jo**
**Tokyo 160-8515 (JP)**

(74) Representative: **Grünecker Patent- und**
**Rechtsanwälte**
**PartG mbB**
**Leopoldstraße 4**
**80802 München (DE)**

(54) **SURGICAL DEVICE**

(57)    The present invention provides a microfracture device and a design method thereof, which are configured to be operable by one person, can ensure a degree of freedom in an angular range of a tip end, and can reliably transmit an external force in a puncture direction while avoiding the occurrence of rotation. The microfracture device includes a needle portion 100, a first support portion 110 connected to the needle portion 100, a second support portion 120 connected to the first support portion 110, and an action portion 130 provided on the second support portion 120 to receive an external force, which are integrally formed.

FIG.1A

## Description

Technical Field

The present invention relates to a microfracture device used in a microfracture method, a surgical device, and a design method of the microfracture device.

Background Art

In the related art, in a surgical instrument for microfracture performed on partial defects of articular cartilage in knee joints, shoulders, hands, feet, and the like, when a plurality of holes is formed in a cartilage surface under an arthroscope, an apparatus is used in which a needle provided at a tip end of an insertion portion, which is inserted through a small incision, has a certain angle with respect to a long axis of the insertion portion in order to form holes substantially perpendicular to the cartilage surface. However, when a force is applied by striking a proximal portion of the insertion portion with a hammer from outside the body, the force is applied in a longitudinal direction of the insertion portion rather than a direction of the needle, so that it is difficult to form the holes substantially perpendicular to the cartilage surface.

For example, as such type of apparatus, as described in the following Patent Literature 1, a device is known, which includes an elongated member, a tip pick, and at least one engaging mechanism for engaging with a complementary-shaped mechanism of a strike apparatus and strikes an impact surface of the strike apparatus to generate a force that is converted by the tip end via the elongated member of the microfracture pick.

In addition, as described in the following Patent Literature 2, a device is known, which includes a microfracture pick, a strike plate, and a punch extension, in which the punch extension is configured to apply an off-axis impact force to the microfracture pick.

Further, as described in the following Patent Literature 3, a device is known, which has tip pick as a double structure of a cannular-shaped outer needle and an inner needle inserted into the outer needle, in which the inner needle protrudes from the curved outer needle.

In addition, as described in the following Patent Literature 4, a device is known, which has a tip pick as a double structure of a cannular-shaped outer needle and an inner needle inserted into the outer needle, in which the inner needle protruding from the curved outer needle is rotated and drilled.

Citation List

Patent Literatures

Patent Literature 1: PCT Japanese Translation Patent Publication No. 2016-511099
Patent Literature 2: U.S. Patent Application Publication No. 2021/386432
Patent Literature 3: U.S. Patent Application Publication No. 2021/177436
Patent Literature 4: U.S. Patent Application Publication No. 2010/249786

Summary of Invention

Technical problem

However, since the devices of Patent Literatures 1 and 2 require the operation of three apparatuses: a microfracture apparatus, a hammer, and a means (such as a hammer or a hand) for striking with the hammer, there is a problem that the operation cannot be performed by one person.

Further, when an impact is not correctly applied in a direction parallel to a direction of the tip end of the device when the impact surface of the strike portion is struck, rotation is applied to the elongated member to cause a force that is not parallel to the direction of the tip end of the hammer device, so that a force and a moment in a direction different from the tip end direction are applied, resulting in problems such as a perforation not being straight, a perforation not being deep, and the tip end being easily damaged.

On the other hand, the devices of Patent Literatures 3 and 4 have a problem in that the structures thereof are complicated, an angle of a tip end portion is limited to 30° or at most 45°, resulting in limitation of a site where the medical treatment can be performed.

In view of the above circumstances, an object of the present invention is to provide a microfracture device, a surgical device, and a design method of the microfracture device, which are configured to be operable by one person, can ensure a degree of freedom in an angular range of a tip end, and can reliably transmit an external force in a puncture direction or a push-in direction while avoiding the occurrence of rotation.

Solution to Problem

A microfracture device of a first aspect is a microfracture device used in a microfracture method, and includes: a needle portion for puncturing; an action portion having an action surface for applying an external force in a puncture direction of the needle portion; a first support portion having a predetermined angle with respect to the puncture direction of the needle portion and connected to the needle portion; and a second support portion having one end with the action portion and the other end connected to the first support portion, in which the needle portion, the action portion, the first support portion, and the second support portion are integrally formed.

According to the microfracture device of the first aspect, since the needle portion, the action portion, the first support portion, and the second support portion are integrally formed, an operator can operate the microfracture device alone.

Further, since the first support portion can set any predetermined angle with respect to the puncture direction of the needle portion, a degree of freedom in an angular range of the tip end can be ensured.

**In** this case, compared to the first support portion, the second support portion having the action portion is configured such that the external force applied to the action surface is directed in the puncture direction of the needle portion. As a result, the external force can be reliably transmitted in the puncture direction while avoiding the occurrence of rotation.

Accordingly, the microfracture device of the first aspect is configured to be operable by one person, can ensure the degree of freedom in the angular range of the tip end, and can reliably transmit the external force in the puncture direction while avoiding the occurrence of rotation.

In the microfracture device of a second aspect, according to the first aspect, the action portion is provided on an extension line of the needle portion in the puncture direction via the second support portion.

According to the microfracture device of the second aspect, the action portion is disposed on the extension line (including substantially on the extension) of the needle portion in the puncture direction, so that the external force applied to the action surface is directed in the puncture direction of the needle portion. Accordingly, it is possible to avoid the occurrence of rotation around the needle portion.

As described above, the microfracture device of the second aspect is configured to be operable by one person, can ensure the degree of freedom in the angular range of the tip end, and can reliably transmit the external force in the puncture direction while avoiding the occurrence of rotation around the needle portion.

In the microfracture device of a third aspect, according to the first aspect, the second support portion extends from the first support portion in the puncture direction of the needle portion, and an end surface of the second support portion opposite to the first support portion is the action surface.

According to the microfracture device of the third aspect, since the second support portion is provided while extending from the first support portion in parallel to the puncture direction of the needle portion, a degree of freedom in shape design can be increased.

In this case, since the external force applied to the action surface is configured to be directed in the puncture direction of the needle portion, the operator can appropriately grip the first support portion or the second support portion, so that it is possible to reliably transmit the external force in the puncture direction while avoiding the occurrence of rotation.

As described above, the microfracture device of the third aspect is configured to be operable by one person, can ensure the degree of freedom in the angular range of the tip end and the degree of freedom in shape design, and can reliably transmit the external force in the puncture direction while avoiding the occurrence of rotation.

**In** the microfracture device of a fourth aspect, according to the third aspect, the first support portion extends beyond the second support portion, and has a grip portion at an end portion of the first support portion opposite to the needle portion.

According to the microfracture device of the fourth aspect, the occurrence of rotation can be avoided by the operator who appropriately grips the first support portion or the second support portion, but the grip portion is provided by extending the first support portion beyond the second support portion, so that it is possible to reliably avoid the occurrence of rotation by allowing the operator to grip the first support portion via the grip portion.

**In** this case, when the external force is applied to the action portion, three rotations of the microfracture device may be generated: (1) rotation around the center of gravity, (2) pitch and yaw rotation of the needle portion, and (3) rotation around an axis of the first support portion.

(1) Rotation around the center of gravity can be avoided by designing such that the external force applied via the action portion passes through the center of gravity. However, when the center of gravity is applied to the microfracture method, the center of gravity may be located in an internal body region where a medical treatment is expected (some adjustment by a length of the second support portion is possible, but there is a possibility that a compact device or the like may be caught on the internal body region), but the first support portion can extend beyond a connection position with the second support portion, thereby moving the center-of-gravity position to an external body region (opposite to the needle portion). Further, a grip portion having a certain amount of mass is provided at an end portion of the first support portion opposite to the needle portion, so that the center-of-gravity position can move to the external body region (opposite to the needle portion).

Accordingly, (1) rotation around the center of gravity can be avoided by moving the center of gravity to the external body region (opposite to the needle portion) and designing such that the external force applied via the action portion reliably passes through the center of gravity.

(2) Pitch and yaw of the needle portion and (3) rotation around the axis of the first support portion can be reliably avoided by providing the grip portion to allow the operator to appropriately grip the grip portion.

As described above, the microfracture device of the fourth aspect is configured to be operable by one person, can ensure the degree of freedom in the angular range of the tip end and the degree of freedom in shape design, and can reliably transmit the external force in the puncture direction while avoiding various rotations.

In the microfracture device of a fifth aspect, according to any one of the first to fourth aspects, the first support portion or the second support portion includes a removal action portion that has a removal action surface for applying a removal force in a direction opposite to the puncture direction of the needle portion.

According to the microfracture device of the fifth aspect, since the removal action portion having the removal action surface for applying the removal force in the direction opposite to the puncture direction of the needle portion is provided, it is possible to avoid a force that is not in parallel to the puncture direction from being applied during removal.

The microfracture device of the fifth aspect is configured to be operable by one person, can ensure the degree of freedom in the angular range of the tip end, can reliably transmit the external force in the puncture direction while avoiding rotation, and can reliably transmit the removal force in the direction opposite to the puncture direction during removal.

In the microfracture device of a sixth aspect, according to the first or second aspect, an extension portion extends between the second support portion and the action portion, the extension portion being inserted into a groove portion formed in a tool that applies a striking force to the action portion.

According to the microfracture device of the sixth aspect, when the needle is removed from the puncture target site, the extension portion is inserted into the groove portion of the tool, and the tool pushes up the surface of the action portion (removal action surface) opposite to the action surface, so that the needle can be easily removed from the puncture target site.

That is, the needle can be removed using the tool while preventing the needle from being removed by rotating the needle. Therefore, it is possible to prevent deformation of the needle, expansion of an inner circumference of the puncture target site, and the like.

In the microfracture device of a seventh aspect, according to the first or second aspect, the needle portion has a needle that punctures a puncture target site, and the needle has a tapered shape that is gradually tapered from a base end side toward a tip end side.

According to the microfracture device according to the seventh aspect, since the needle has a tapered shape that is gradually tapered from the base end side toward the tip end side, it is possible to maintain rigidity of the base end (root) side of the needle and easily puncture the puncture target site while suppressing a puncture resistance when the needle punctures the puncture target site.

In addition, when the needle is removed from the puncture target site, the needle can be easily removed from the puncture target site while suppressing the bending of the needle or the like.

A surgical device of a joint part of an eighth aspect includes: a device portion pushed into a predetermined site of the joint part to perform a medical treatment on the predetermined site; an action portion having an action surface for applying an external force in a push-in direction of the device portion; a first support portion having a predetermined angle with respect to the push-in direction of the device portion and connected to the device portion; and a second support portion having one end with the action portion and the other end connected to the first support portion, in which the device portion, the action portion, the first support portion, and the second support portion are integrally formed.

According to the surgical device for the joint part according to the eighth aspect, since the device portion, the action portion, the first support portion, and the second support portion are integrally formed, the operator can operate the surgical device alone.

Further, since the first support portion can set any predetermined angle with respect to the push-in direction of the device portion, a degree of freedom in an angular range of the tip end can be ensured.

In this case, the second support portion having the action portion is configured such that the external force applied to the action surface is directed in the push-in direction of the device portion with respect to the first support portion. As a result, the external force can be reliably transmitted in the push-in direction while avoiding the occurrence of rotation.

Accordingly, the surgical device of the joint part the eighth aspect is configured to be operable by one person, can ensure the degree of freedom in the angular range of the tip end, and can reliably transmit the external force in the push-in direction while avoiding the occurrence of rotation.

In the surgical device of a joint part of a ninth aspect, according to the eighth aspect, the first support portion extends by a predetermined length, and a handle portion is provided while extending by a predetermined length from a connection part between the first support portion and the second support portion in a direction opposite to an extension direction of the first support portion.

According to the above-described aspect, since the device portion can be operated by a device user who grips the handle portion and appropriately operates the handle portion, it is possible to support a medical treatment work on the predetermined site by the device portion, and it is possible to improve simplicity, stability, accuracy, or the like of the medical treatment using the surgical device.

In the surgical device of a joint part of a tenth aspect, according to the eighth aspect, the first support portion extends by a predetermined length, and a handle portion does not extend from a connection part between the first support portion and the second support portion.

A design method of a microfracture device of an eleventh aspect is a method for designing the microfracture device of the fourth aspect, and includes: a center-of-gravity calculation step of calculating a center of gravity of the microfracture device; a provisional determination step of provisionally determining the action portion such that the external force applied via the action surface passes through the center of gravity; a verification step of verifying whether or not the action portion, which has been provisionally determined by the provisional determination step, is located in an internal body region where a medical treatment is expected, when the microfracture method is applied; and an action position determination step of determining a position where the center of gravity and the action portion are moved to an external body region as an action position of the action portion by adjusting at least a mass of the grip portion, when the action portion is located in the internal body region by the verification step.

According to the design method of the microfracture device of the eleventh aspect, among (1) rotation around the center of gravity, (2) two rotations of pitch and yaw of the needle portion, and (3) rotation around an axis of first support portion, which may be generated in the microfracture device when the external force is applied to the action portion, in particular, a design of avoiding (1) rotation around the center of gravity is possible.

That is, the rotation around the center of gravity can be avoided by designing such that the external force applied via the action portion passes through the center of gravity, but the center of gravity may be located in the internal body region where a medical treatment is expected, when the center of gravity is applied to the microfracture method.

Therefore, after the action portion is provisionally determined such that the external force passes through the center of gravity of the microfracture device, which is calculated by the center-of-gravity calculation step, it is possible to verify whether or not the provisionally determined action portion is located in the internal body region where a medical treatment is expected.

When the action portion is located in the internal body region by the verification step, it is possible to determine the position where the center of gravity and the action portion are moved to the external body region as an action position of the action portion by adjusting the mass of the grip portion.

Accordingly, the design method of the microfracture device of the eleventh aspect is configured to be operable by one person, can ensure the degree of freedom in the angular range of the tip end and the degree of freedom in shape design, and can reliably transmit the external force in the puncture direction while avoiding various rotations.

In the design method of the microfracture device of a twelfth aspect, according to the eleventh aspect, the design method further includes: an action surface shape determination step of determining a shape of the action surface by evaluating a deviation of a traveling direction of the needle portion according to a position of the action surface where the external force is applied.

According to the design method of the microfracture device of the twelfth aspect, since the action surface has a certain area, the deviation in the traveling direction at the action position of the external force can be evaluated, thereby determining the shape such that the deviation is minimized, for example.

Accordingly, the design method of the microfracture device of the twelfth aspect is configured to be operable by one person, can ensure the degree of freedom in the angular range of the tip end and the degree of freedom in shape design, and can reliably transmit the external force in the puncture direction while avoiding various rotations and deviations.

Brief Description of Drawings

FIG. 1A is a perspective view illustrating a usage state of an example of a microfracture device according to a first embodiment.

FIG. 1B is a perspective view illustrating a usage state of an example of the microfracture device of the present embodiment.

FIG. 2 is an explanatory view illustrating an angle variation of the microfracture device of FIG. 1.

FIG. 3 is an explanatory view illustrating a variation of a needle portion of the microfracture device of FIG. 1.

FIG. 4A is a perspective view illustrating a usage state of another example of the microfracture device of a second embodiment.

FIG. 4B is a perspective view illustrating a usage state of another example of the microfracture device according to the present embodiment.

FIG. 5 is an explanatory view illustrating an angle variation of the microfracture device of FIG. 4.

FIG. 6 is an explanatory view illustrating an angle variation of the microfracture device of FIG. 4.

FIG. 7 is an explanatory view illustrating a design method of the microfracture device of FIG. 4.

FIG. 8 is an explanatory view illustrating details of the design method of the microfracture device of FIG. 7.

FIG. 9 is an explanatory view illustrating details of the design method of the microfracture device of FIG. 7.

FIG. 10 is an explanatory views illustrating details of the design method of the microfracture device of FIG. 9.

FIG. 11A is an explanatory view illustrating the design method of the microfracture device of FIG. 4.

FIG. 11B is an explanatory view illustrating the design method of the microfracture device of FIG. 4.

FIG. 12A is an explanatory view illustrating a configuration of a needle portion of the microfracture device of FIG. 1 and FIG. 4

FIG. 12B is an explanatory view illustrating another configuration of the needle portion of the microfracture device of FIG. 1 and FIG. 4.

FIG. 13 is a perspective view of a microfracture device according to a third embodiment.

FIG. 14 is a perspective view and a side view of a needle of the microfracture device of the embodiment, respectively.

FIG. 15 is an explanatory view illustrating a first embodiment of a surgical device of a joint part and is an explanatory view illustrating a first usage example thereof.

FIGS. 16 is an explanatory views illustrating a second usage example of the first embodiment.

FIG. 17 is a perspective view illustrating an application site and the like of the first embodiment.

FIG. 18 is a perspective view illustrating a second embodiment of a surgical device of a joint part.

FIG. 19 is a perspective view illustrating a third embodiment of a surgical device of a joint part.

FIG. 20 is a perspective view illustrating a fourth embodiment of a surgical device of a joint part.

FIG. 21 is a perspective view illustrating a fifth embodiment of a surgical device of a joint part.

FIG. 22 is an explanatory view illustrating a sixth embodiment of a surgical device of a joint part.

FIG. 23 is a perspective view illustrating a usage example of the sixth embodiment.

FIG. 24 is an explanatory view illustrating a seventh embodiment of a surgical device of a joint part.

FIG. 25 is a perspective view illustrating a usage example of the seventh embodiment.

Description of Embodiments

(First Embodiment of Microfracture Device)

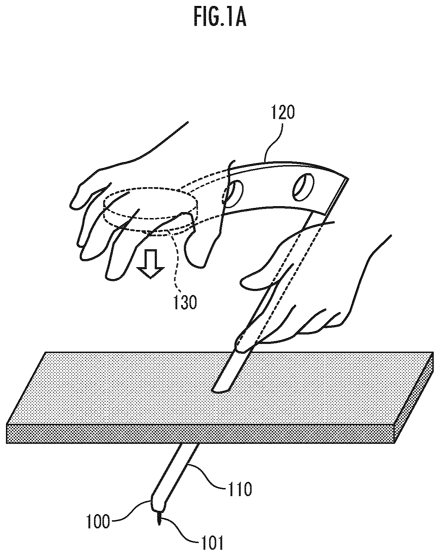

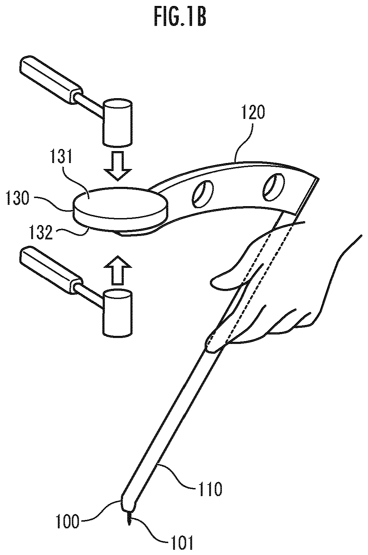

As illustrated in FIG. 1, a microfracture device as a first embodiment includes a needle portion 100, a first support portion 110 connected to the needle portion 100, a second support portion 120 connected to the first support portion 110, and an action portion 130 provided on the second support portion 120 and receiving an external force, and is used in a microfracture method.

The needle portion 100 has a pick-shaped needle 101 for puncturing, and supports the needle 101 on one end side of the first support portion 110.

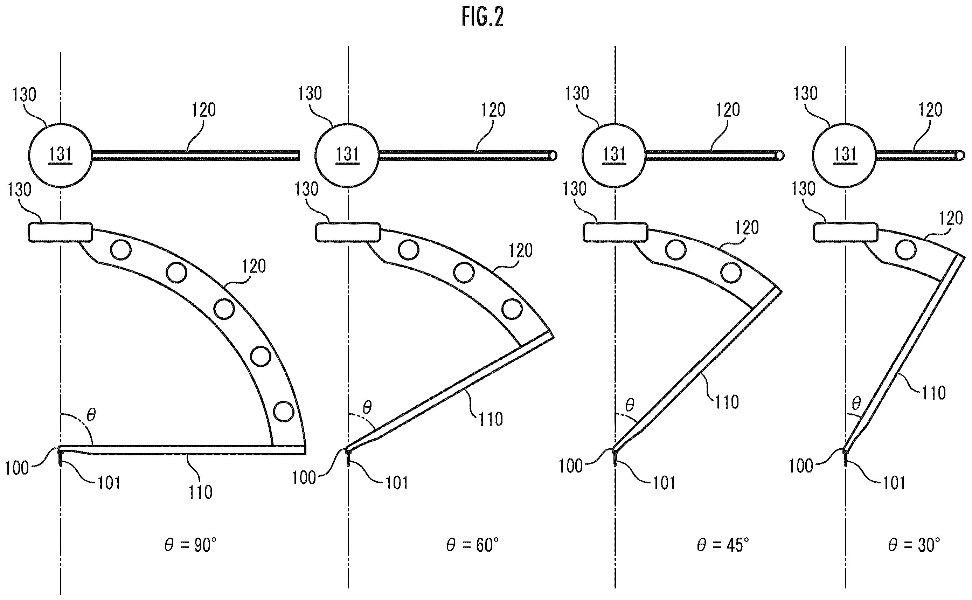

The first support portion 110 is a rigid body (in the present embodiment, a rod body) that extends from the needle portion at a predetermined angle θ (see FIG. 2) with respect to a puncture direction of the needle portion 100.

The second support portion 120 is an arch-shaped (circular arc-shaped) rigid body (in the present embodiment, a plate body) having one end connected to the first support portion and the other end provided with an action portion.

The action portion 130 has a disc shape in which an action surface 131 for applying an external force in the puncture direction and a removal action surface 132 for applying a removal force for removal form front and back sides, and is disposed on a substantial extension line of the needle portion 100 in the puncture direction.

In the microfracture device configured as described above, the needle portion 100, the first support portion 110, the second support portion 120, and the action portion 130 are integrally formed.

Therefore, as illustrated in FIG. 1A, for example, the operator can puncture a desired site with the needle 101 of the needle portion 100 by applying the external force to the action surface 131 of the action portion 130 with one hand while supporting the first support portion 110 with the other hand.

In addition, as illustrated in FIG. 1B, even when a tool (for example, a hammer) is used to apply a striking force, the operator can puncture the desired site with the needle 101 of the needle portion 100 by gripping the tool with one hand while supporting the first support portion 110 with the other hand, and striking the action surface 131 of the action portion 130 using the tool to apply the external force.

In this case, as illustrated in FIG. 2, when an angle formed with respect to the puncture direction (vertical direction in FIG. 2) of the needle portion 100 is defined as θ, the first support portion 110 has a plurality of variations of angles, such as θ = 90°, θ = 60°, θ = 45°, and θ = 30°, according to an approach to an application location.

In any case of θ = 90°, θ = 60°, θ = 45°, and θ = 30° in FIG. 2, the action portion 130 (action surface 131) is disposed on the substantial extension line of the needle portion 100 in the puncture direction. More specifically, a puncture axis of the needle 101 of the needle portion 100 is at the center of the disc-shaped action surface 131.

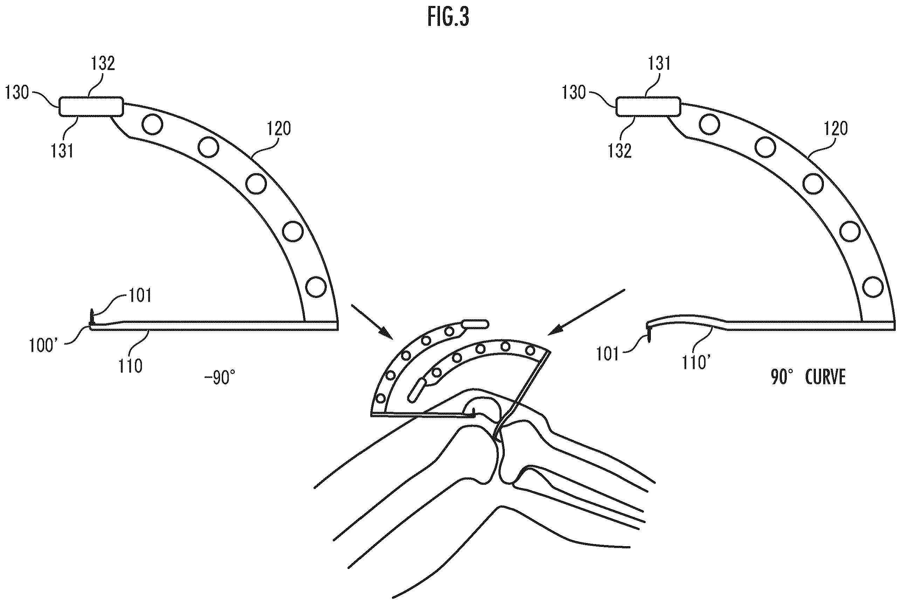

Further, as illustrated in FIG. 3, a direction of the needle 101 of the needle portion 100' may be disposed at -90° as in the left side in FIG. 3. Accordingly, the needle portion 100' is disposed in such a manner that the needle 101 faces upward, which can also be applied to a case where the microfracture method is performed on cartilage from below, such as a patella.

In addition, as illustrated on the right side in FIG. 3, a first support portion 110' may be curved toward the needle portion 100 side. Accordingly, the first support portion 110' is curved toward the needle portion 100 side, which can also be applied to a case where the microfracture method is performed on the cartilage in a narrow space of a proximal portion of the tibia.

As described in detail above, according to the microfracture device of the first embodiment, since the needle portion 100, the first support portion 110, the second support portion 120, and the action portion 130 are integrally formed, the operator can operate the device alone.

In addition, since the first support portion 110 can set any predetermined angle with respect to the puncture direction of the needle portion 100 (see FIG. 2), a degree of freedom in the angular range of the tip end can be ensured.

Further, the action portion 130 is disposed on the substantial extension line of the needle portion 100 in the puncture direction (in the present embodiment, in particular, directly above the needle portion 100, which is on the extension line), so that the external force applied to the action surface 131 is directed in the puncture direction of the needle portion. Accordingly, it is possible to avoid the occurrence of rotation around the needle portion 100.

Similarly, since the removal force applied to the removal action surface 132 is also applied in a direction opposite to the puncture direction on the puncture axis, a circumferential portion is not damaged during removal.

Accordingly, the microfracture device of the first embodiment is configured to be operable by one person, can ensure the degree of freedom in the angular range of the tip end, and can reliably transmit the external force in the puncture direction while avoiding the occurrence of rotation.

(Second Embodiment of Microfracture Device)

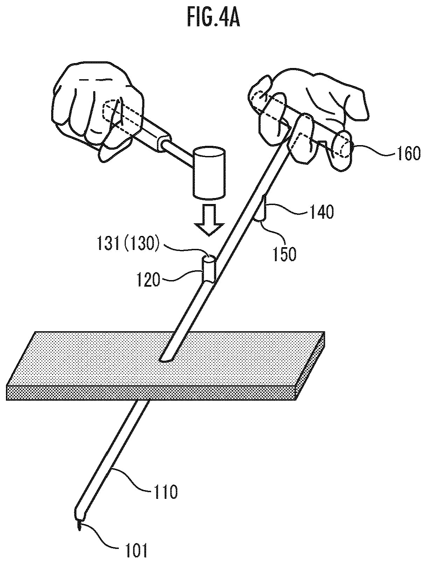

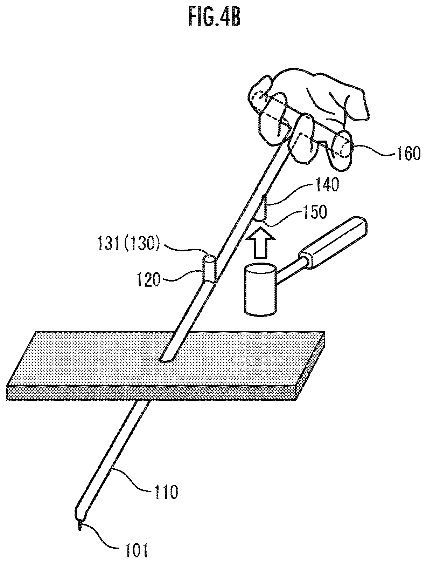

Next, a microfracture device according to a second embodiment will be described with reference to FIG. 4. The same reference numerals are assigned to the same configurations as those in the first embodiment, and the description thereof will be omitted.

In the microfracture device according to the second embodiment, a second support portion 120 is a rod-shaped rigid body extending from a first support portion 110 in a puncture direction of a needle portion 100, and an end surface of the second support portion 120 opposite to the first support portion 110 is an action surface 131. In addition, the action surface 131 is a flat surface substantially perpendicular to the puncture direction of the needle portion 100.

In addition, a removal action portion 140, which is a rod-shaped rigid body, extends in the puncture direction to face the second support portion 120 via the first support portion 110, and an end surface of the removal action portion 140 is a removal action surface 150.

Further, the first support portion 110 extends beyond the second support portion 120 and has a handle-shaped (in the present embodiment, T-shaped handle) grip portion 160 at an end portion of the first support portion 110 opposite to the needle portion 100.

In the microfracture device of the second embodiment configured as described above, the needle portion 100, the first support portion 110, the second support portion 120, and the action portion 130 are integrally formed.

Therefore, as illustrated in FIG. 4A, for example, the operator can puncture a desired site with the needle 101 of the needle portion 100 by gripping a tool (for example, a hammer) that applies a striking force with one hand while gripping the grip portion 160 with the other hand to apply an external force to the action surface 131 using the tool.

In addition, as illustrated in FIG. 4B, even in a case of removal, the operator can remove the needle 101 of the needle portion 100 in a direction opposite to the puncture direction by gripping a tool (for example, a hammer) that applies the striking force with one hand while gripping the grip portion 160 with the other hand to apply a removal force to the removal action surface 150 using the tool.

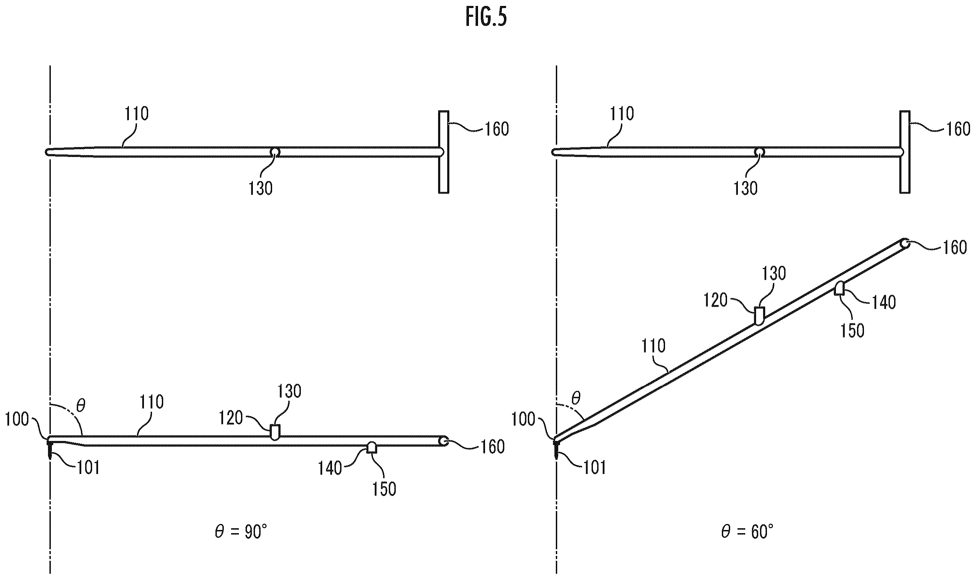

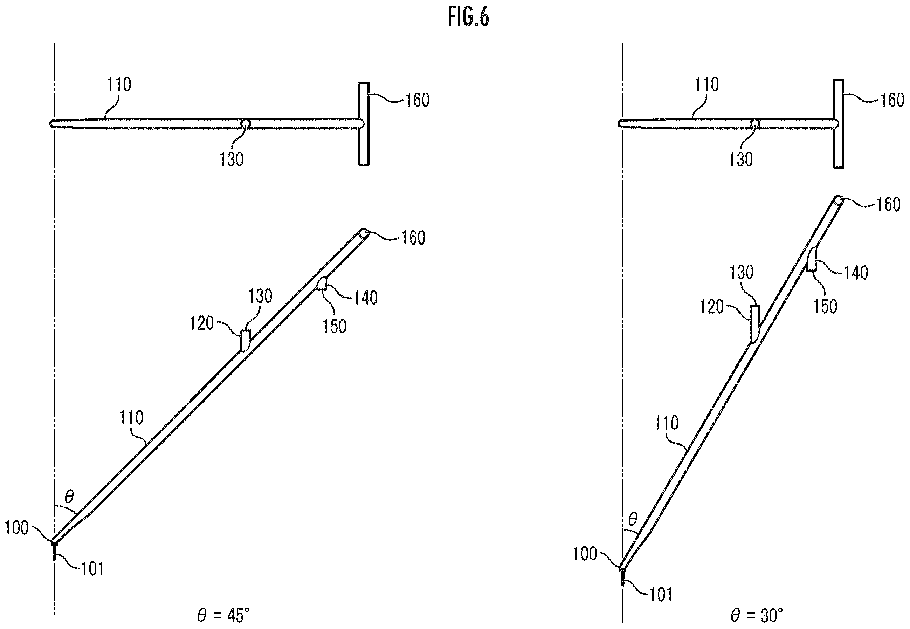

In this case, as illustrated in FIGS. 5 and 6, when an angle formed with respect to the puncture direction (vertical direction in FIGS. 5 and 6) of the needle portion 100 is defined as θ, the first support portion 110 has a plurality of variations of angles, such as θ = 90°, θ = 60°, θ = 45°, and θ = 30°, according to an approach to an application location.

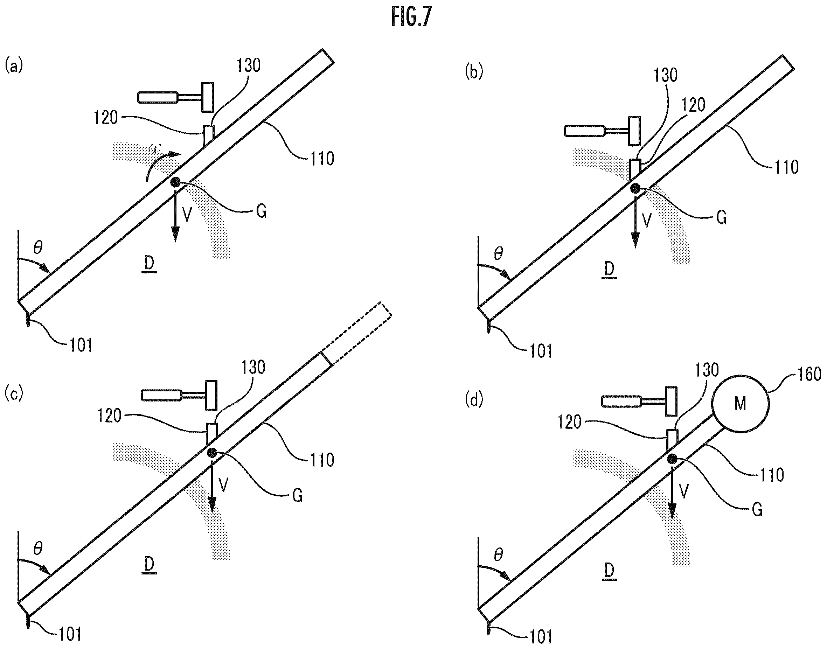

Next, a design method of the microfracture device according to the second embodiment will be described with reference to FIG. 7.

First, as illustrated in FIG. 7(a), when a center-of-gravity position of the device is defined as G, the device rotates around a center of gravity at an angular velocity ω when the external force that is applied via the action portion 130 does not pass through the center-of-gravity position G.

Therefore, as illustrated in FIG. 7(b), when the second support portion 120 is simply provided to pass through the center-of-gravity position G, the external force passes through the center-of-gravity position G via the action portion 130, but the second support portion 120 or the action portion 130 is caught on an internal body region D where a medical treatment is expected.

On the other hand, as illustrated in FIG. 7(c), it is also considered that the center-of-gravity position G moves outside the internal body region D by largely extending the first support portion 110 in a direction opposite to the needle portion 100, but in this case, the device itself becomes longer.

Therefore, in the present embodiment, as illustrated in FIG. 7(d), a mass M of the grip portion 160 is adjusted (instead of largely extending the first support portion 110 in the direction opposite to the needle portion 100) to design the device itself in a compact manner.

Based on the design concept, in the design method of the microfracture device of the second embodiment, (1) first, a center-of-gravity calculation step of calculating the center-of-gravity position G of the microfracture device is performed, (2) next, a provisional determination step of provisionally determining the action portion 130 such that the external force applied via the action surface 131 of the action portion 130 passes through the center-of-gravity position G is performed, and (3) a verification step of verifying whether or not the action portion 130, which has been provisionally determined by the provisional determination step, is located in the internal body region D where a medical treatment is expected, when the microfracture method is applied is performed.

In addition, (4) an action position determination step of determining a position where the center-of-gravity position G and the action portion 130 (including the second support portion 120) are moved to an external body region as an action position of the action portion 130 by adjusting (increasing) at least a mass M of the grip portion 160, when the action portion 130 is located in the internal body region D by the verification step, is performed.

In the action position determination step, when the action portion 130 is not located in the internal body region D in the verification step, the action portion 130 (including the second support portion 120), which passes through the center-of-gravity position G that has been provisionally determined in the provisional determination step, may be determined as the action position of the action portion 130 as it is.

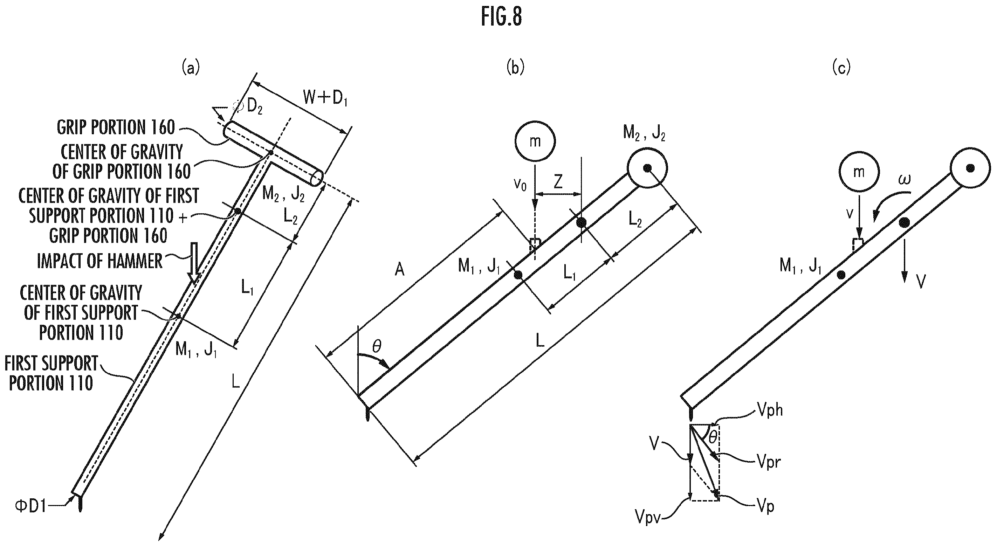

More specifically, as illustrated in FIG. 8(a), first, in the simulation, a model is formed with a configuration including a cylindrical first support portion 110 and a cylindrical grip portion 160 provided perpendicular to an end portion of the first support portion 110, and masses $M_1$ and $M_2$ and inertia moments $J_1$ and $J_2$ of the first support portion 110 and the grip portion 160 are obtained, respectively.

[Equation 1]

$$M_1 = \frac{1}{4}\rho D_1{}^2 L$$

[Equation 2]

$$M_2 = \frac{1}{4}\rho D_2{}^2 W$$

[Equation 3]

$$J_1 = \frac{1}{3}M_1((L - L_2)^2 - (L - L_2)L_2 + L_2{}^2)$$

[Equation 4]

$$J_2 = \frac{1}{8}M_2(D_2{}^2 + 8L_2{}^2)$$

The characters in Equations 1 to 4 and the drawings mean as follows.

$J_1$: Inertia moment of first support portion 110 around first support portion 110 + grip portion 160
$M_1$: Mass of first support portion 110
L: Length of first support portion 110
$L_1$: Distance between center of gravity of first support portion 110 and center of gravity of first support portion 110 + grip portion 160
$D_1$: Outer diameter of first support portion 110
$J_2$: Inertia moment of support portion around first support portion 110 + grip portion 160
$M_2$: Mass of grip portion 160
$D_2$: Outer diameter of grip portion 160
$L_2$: Distance between center of gravity of grip portion 160 and center of gravity of first support portion 110 + grip portion 160
P: Specific gravity of first support portion 110 and grip portion 160 (SUS304 7.93)
W: Length of grip portion 160

For the model illustrated in FIG. 8(b), the angular velocity $\omega$ around the center of gravity illustrated in FIG. 8(c) is derived as in the following Equation 8 from the law of conservation of momentum of the following Equation 5, the law of conservation of angular momentum around the center of gravity of Equation 6, and the law of conservation of energy of Equation 7.

[Equation 5]

$$mv_0 = (M_1 + M_2)V - mv$$

[Equation 6]

$$mv_0Z = (J_1 + J_2)\,\omega - mv \cdot Z$$

[Equation 7]

$$\frac{1}{2}mv_0{}^2 = \frac{1}{2}(M_1 + M_2)V^2 + \frac{1}{2}(J_1 + J_2)\,\omega^2 + \frac{1}{2}mv^2$$

[Equation 8]

$$\omega = \frac{2\,v_0}{\dfrac{J_1 + J_2}{mZ} + \dfrac{J_1 + J_2}{(M_1 + M_2)Z} + Z}$$

A relational equation of L, $L_1$, and $L_2$ is as shown in the following Equation 9, and a relational equation of Vp is as shown in the following Equation 10.

[Equation 9]

$$L_1 = \frac{M_2}{M_1 + M_2} \cdot \frac{L}{2}$$

$$L_2 = \frac{M_1}{M_1 + M_2} \cdot \frac{L}{2}$$

$$Z = \cos\theta(L - L_2 - A)$$

[Equation 10]

$$Vpr = (L - L_2)\omega$$

$$Vph = Vpr \cdot \cos\theta$$

$$Vpv = V + Vpr \cdot \sin\theta$$

$$Vp = \sqrt{Vph^2 + Vpv^2}$$

The characters in Equations 5 to 10 and the drawings mean as follows.

$\theta$: Angle between first support portion 110 and needle 101
m: Mass of head of hammer
$v_0$: Velocity of hammer upon impact
v: Velocity of hammer after impact
A: Distance from tip end of first support portion 110 to impact portion of hammer
Z: Horizontal distance from impact portion of first support portion 110 to center of gravity of first support portion 110 + grip portion 160
$J_1$: Inertia moment of first support portion 110 around first support portion 110 + grip portion 160
$M_1$: Mass of first support portion 110
L: Length of first support portion 110
$L_1$: Distance between center of gravity of first support portion 110 and center of gravity of first support portion 110 + grip portion 160
$J_2$: Inertia moment of support portion around first support portion 110 + grip portion 160
$M_2$: Mass of grip portion 160
$D_2$: Outer diameter of grip portion 160
$L_2$: Distance between center of gravity of grip portion 160 and center of gravity of first support portion 110 + grip portion 160
V: Velocity of center of gravity of first support portion 110 + grip portion 160 after impact
$\omega$: Rotational angular velocity of first support portion 110 + grip portion 160 around center of gravity after impact
Vp: Velocity vector of needle 101 after impact
Vpv: Vertical component in direction of needle 101 at velocity of needle 101 after impact
Vph: Parallel component in direction of needle 101 at velocity of needle 101 after impact

In this case, for the sake of convenience, the derivation of Equation 8 from Equations 5 to 7 will be described below.

First, Equations 5 to 7 are transformed into following (A) to (C), respectively.

[Equation 11]

$$mv_0 = (M_1 + M_2)V - mv$$

$$\therefore \quad v_0 = \frac{M_1 + M_2}{m}V - v \quad \cdot \cdot (A)$$

$$mv_0 Z = (J_1 + J_2)\,\omega - mv \cdot Z$$

$$\therefore \quad v_0 = \frac{J_1 + J_2}{mZ}\omega - v \quad \cdot \cdot (B)$$

$$\frac{1}{2}mv_0{}^2 = \frac{1}{2}(M_1 + M_2)V^2 + \frac{1}{2}(J_1 + J_2)\,\omega^2 + \frac{1}{2}mv^2$$

$$\therefore \quad v_0{}^2 = \frac{M_1 + M_2}{m}V^2 + \frac{J_1 + J_2}{m}\omega^2 + v^2 \quad \cdot \cdot (C)$$

Next, from Equation (A) and Equation (B),

[Equation 12]

$$V = \frac{J_1 + J_2}{M_1 + M_2} \cdot \frac{1}{Z}\omega$$

By substituting into (C)

[Equation 13]

$$v_0{}^2 - v^2 = \frac{(J_1 + J_2)^2}{m\,(M_1 + M_2)} \cdot \left(\frac{1}{Z}\right)^2 \omega^2 + \frac{J_1 + J_2}{m}\omega^2$$

From (B)

[Equation 14]

$$v_0 + v = \frac{J_1 + J_2}{mZ}\,\omega$$

$$v_0 - v = \frac{J_1 + J_2}{(M_1 + M_2)Z}\,\omega + Z\omega$$

$$2\,v_0 = \frac{J_1 + J_2}{mZ}\,\omega + \frac{J_1 + J_2}{(M_1 + M_2)Z}\,\omega + Z\omega$$

$$\omega = \frac{2\,v_0}{\dfrac{J_1 + J_2}{mZ} + \dfrac{J_1 + J_2}{(M_1 + M_2)Z} + Z}$$

Accordingly, the microfracture device can be designed such that the angular velocity $\omega$ around the center of gravity derived as in Equation 8 is, for example, 0. The expansion of a hole during perforation can be evaluated by a ratio of the velocity Vph of deviating in the horizontal direction to the velocity Vpv of traveling in the direction of the needle 101.

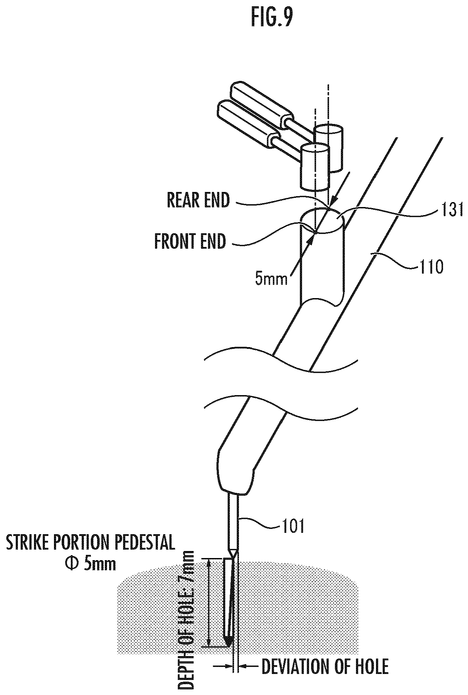

Regarding the evaluation, a design method of a shape of the action surface of the microfracture device according to the second embodiment will be described with reference to FIGS. 9 to 10. More specifically, the shape of the action surface 131 is determined by evaluating the deviation of the needle 101 of the needle portion 100 in a traveling direction according to a position where the external force is applied to the action surface 131.

As schematically illustrated in FIG. 9, the action surface 131, which is provided on the first support portion 110 via the second support portion 120 is a columnar pedestal with a horizontal upper surface, and has an outer diameter of about $\varphi3$ to $\varphi5$ mm.

When the action surface 131 is struck with a hammer, a case of hitting a front end or a rear end of the pedestal is considered as illustrated in FIG. 9, and thus it is possible to calculate how much the hole deviates horizontally in each case.

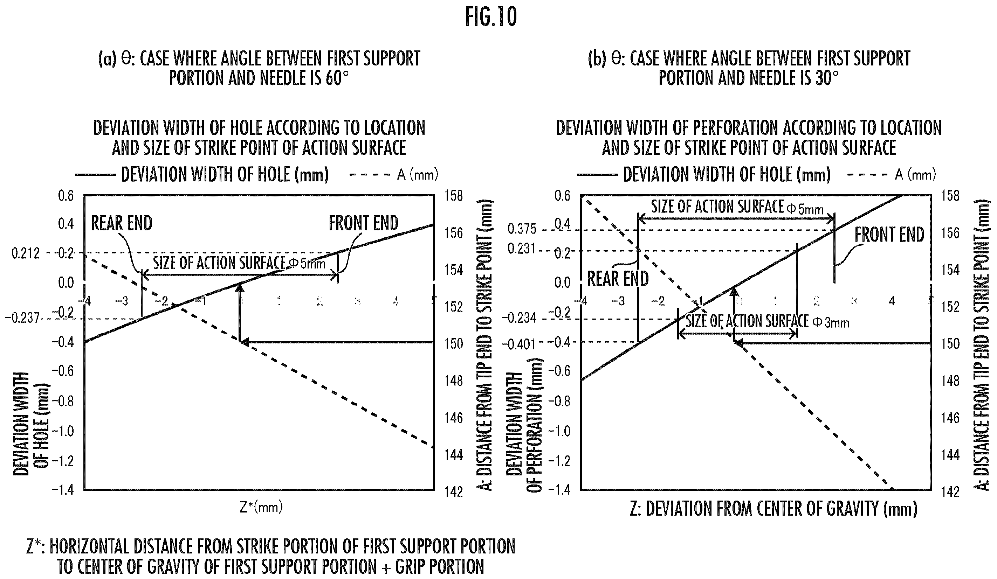

The calculation results are illustrated in FIGS. 10(a) and 10(b). The calculation is performed by obtaining the above-described ratio of Vph/Vpv and multiplying the ratio by a depth of the hole equal to the length of the needle, which is 7 mm.

FIG. 10(a) illustrates a case where an angle $\theta$ between the first support portion 110 and the needle 101 is 60°, and FIG. 10(b) illustrates a case where the angle $\theta$ is 30°.

For the cases, when horizontal deviations of both cases of the pedestals with an outer diameter of $\varphi5$ mm are compared, as can be seen from the fact that Vph = Vpr·cos$\theta$, which is a numerator of the ratio Vph/Vpv, becomes larger as the angle $\theta$ becomes smaller, the smaller the angle $\theta$ of 30°, the larger the horizontal deviation (-0.401 to 0.375 mm). When a thickness of the needle 101 (an inner diameter of the hole) is $\varphi1$ mm, the hole is expanded by about 0.4, so that in order to suppress the expansion, a size of the action surface 131, which is the pedestal, is set to $\varphi3$ mm, and thus the horizontal deviation (-0.234 to 0.231 mm) can be suppressed.

In the simulations of FIGS. 10(a) and 10(b), the following values were used.

$J_1$: 201.85 kgmm$^2$
$M_1$: 0.036 kg
$D_1$: $\varphi5$ mm
L: 230 mm
L1: 35 mm
J2: 100 kgmm$^2$
M2: 0.0157 kg
D2: $\varphi6$ mm
L2: 80 mm

As described in detail above, according to the microfracture device of the second embodiment, since the needle portion 100, the first support portion 110, the second support portion 120, the action portion 130, and the removal action portion 140 (removal action surface 150) are integrally formed, the operator can perform puncture and removal alone.

In addition, since the first support portion 110 can set any predetermined angle with respect to the puncture direction of the needle portion 100 (see FIGS. 5 and 6), a degree of freedom in the angular range of the tip end can be ensured.

Further, in addition to the rotation of the device around the center of gravity (see FIG. 7), it is possible to avoid the occurrence of various rotations that are applied during the puncture.

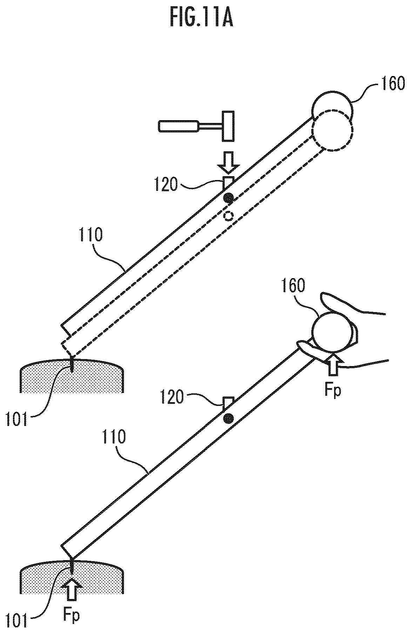

For example, as illustrated in the upper part of FIG. 11A, before the puncture, when the external force is applied to the action portion 130 (action surface 131) using a hammer, the external force passes through the center-of-gravity position G of the device, and thus the device is moved in parallel in the puncture direction.

On the other hand, as illustrated in the lower part of FIG. 11A, after the puncture of the needle 101 of the needle portion 100 is started, a reaction force Fp is applied to the needle 101, but a reaction force that is applied to the grip portion 160 from the hand of the operator is supported to be substantially the same as the reaction force Fp, so that the device can be kept parallel, and the occurrence of rotation (pitch) of the needle portion can be avoided.

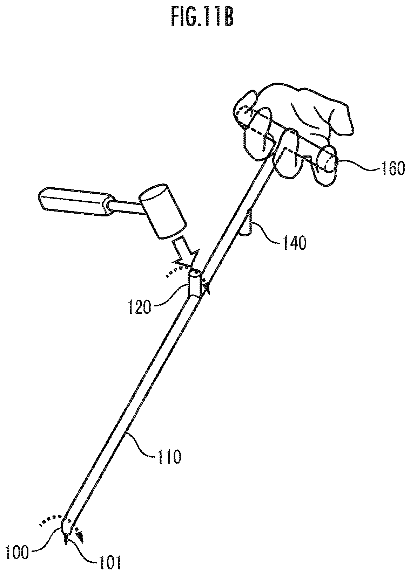

Further, as illustrated in FIG. 11B, although a moment around an axis of the first support portion 110 may act, the grip portion 160 such as a T-shape is provided to intersect the first support portion 110, so that the grip portion 160 can be caught on the finger to prevent the occurrence of rotation, and the occurrence of rotation around the axis of the first support portion 110 can be avoided. In order to suppress the moment of the first support portion 110 around the axis, it is desirable that the length of the second support portion 120 is short.

Accordingly, it is possible to avoid the occurrence of various rotations that may occur in the device and to reliably transmit the external force in the puncture direction.

Accordingly, the microfracture device of the second embodiment is configured to be operable by one person, can ensure the degree of freedom in the angular range of the tip end and the degree of freedom in shape design, and can reliably transmit the external force in the puncture direction while avoiding the occurrence of various rotations.

The respective components of the microfracture devices of the first embodiment and the second embodiment may be appropriately combined. For example, the needle portion 100' (see FIG. 3) in which the needle 101 of the first embodiment faces upward or the shape (see FIG. 3) in which the first support portion 110' is curved toward the needle portion 100 side may be adopted to the microfracture device of the second embodiment.

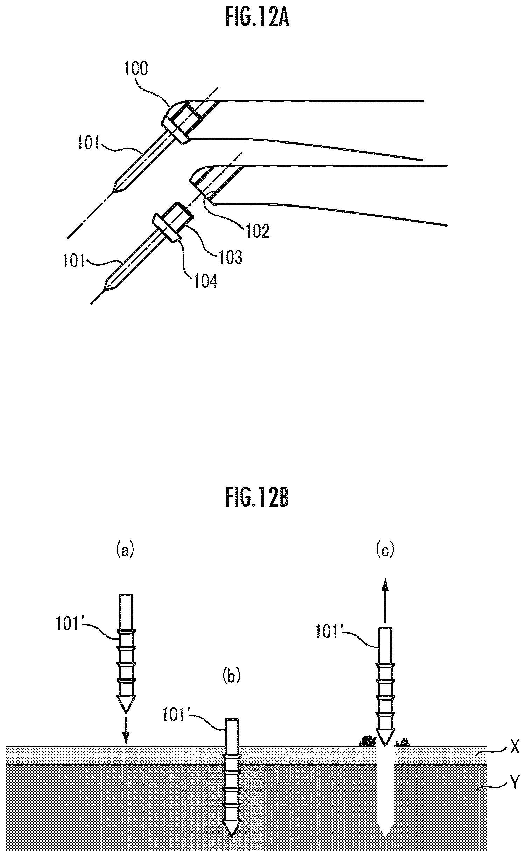

In addition, as illustrated in FIG. 12A, the microfracture devices of the first embodiment and the second embodiment may be configured such that the needle 101 of the needle portion 100 may be detachable. In this case, it is preferable that a fitting portion 102 to which the needle 101 is fitted is provided on the first support portion 110 side, and the needle 101 is provided with a flange portion 104 that abuts together with a fitting surface 103 that is fitted to the fitting portion 102. Accordingly, it is possible to cope with a case where replacement of the needle portion 100 (needle 101) due to deformation, abrasion, or the like is required.

Further, as illustrated in FIG. 12B, by providing a needle 101' having a plurality of circular crown-shaped wedges around the needle 101', when the needle 101' is punctured, more specifically, when the needle 101' is driven into a tissue of a subchondral bone Y beyond a cartilage X, it is possible to suppress compression of the tissue and the crushing of pores between original tissues, and to prevent the bone marrow or the mesenchymal stem cells for regenerating the cartilage from being difficult to pass through the pores, thereby preventing the effect of the microfracture method from being impaired.

In addition, in this case, it is also expected that when the needle 101' is removed, once edges of the wedges scrape off a surface of the compressed tissue by the circular crown-shaped wedges, thereby allowing the pores to communicate with each other.

(Third Embodiment of Microfracture Device)

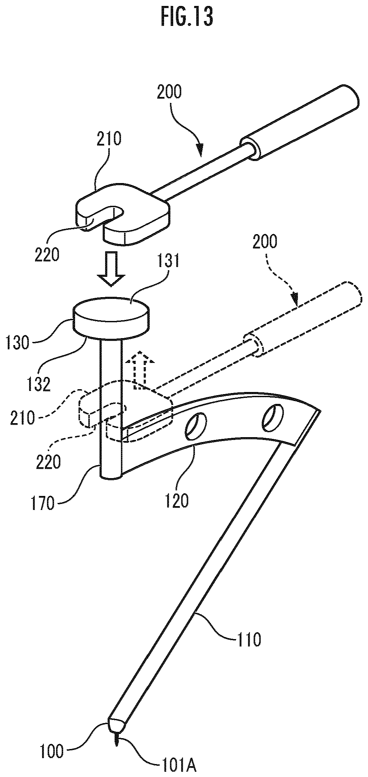

Next, a microfracture device according to a third embodiment will be described with reference to FIG. 13. The same reference numerals are assigned to the same configurations as those in the first and second embodiments, and the description thereof will be omitted.

The microfracture device of the third embodiment has a structure in which an extension portion 170, which is inserted into a groove portion 220 formed in a tool 200 that applies a striking force to the action portion 130, extends between a second support portion 120 and an action portion 130.

More specifically, the tool 200 according to the present embodiment is a so-called hammer having a strike portion 210 that applies a striking force to the action portion 130. In addition, the groove portion 220 having a substantially U-shape is formed by cutting out at a center portion in the width direction of a tip end portion of the strike portion 210.

Further, the extension portion 170 having a substantially circular rod shape extends from a tip end of the second support portion 120 having a substantially circular arc plate shape. The extension portion 170 extends to be located on a substantial extension line with respect to the puncture direction of the needle portion 100. Then, the action portion 130 having a substantially disc shape with a predetermined thickness is connected to a tip end portion of the extension portion 170 in an extension direction. That is, the extension portion 170 is provided between the second support portion 120 and the action portion 130.

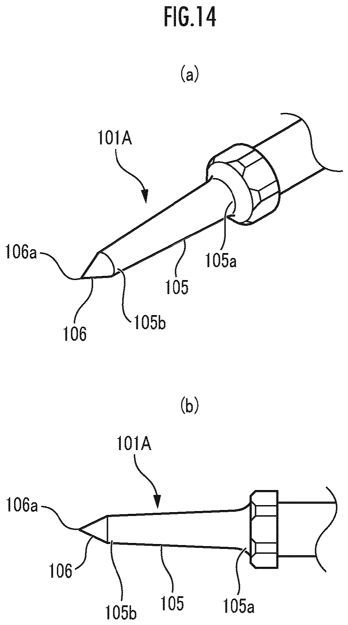

In addition, as illustrated in FIGS. 14(a) and 14(b), in the microfracture device of the third embodiment, a needle 101A that punctures a puncture target site has a tapered shape that is gradually tapered from a base end 105a side toward a tip end (here, an utmost tip end 106a) side.

More specifically, the needle 101A includes a base portion 105 that extends to a predetermined length and a puncture portion 106 that is consecutively provided at a tip end of the base portion 105 in the extension direction and has a substantially conical shape. In addition, the base portion 105 has the base end 105a in the extension direction, which is larger in diameter than the tip end 105b in the extension direction, and an outer circumference of the base portion 105 has an R-shaped curved shape. Further, the puncture portion 106 having a substantially conical shape has a base end side with an increased diameter and the utmost tip end 106a that is pointed, and an outer circumference of the puncture portion 106 has a tapered shape.

That is, an outer circumference of the needle 101A has a tapered shape that is gradually tapered in diameter from the base end 105a side toward the utmost tip end 106a side.

In the microfracture device having the above-described configuration, when the needle 101A punctures the puncture target site, the strike portion 210 strikes the action surface 131 of the action portion 130 to apply an external force, as in the tool 200 indicated by a solid line in FIG. 13. Then, the external force is transmitted to the needle portion 100 via the second support portion 120 and the first support portion 110, and the needle 101A punctures the puncture target site.

On the other hand, when the needle 101A is removed from the puncture target site, first, the extension portion 170 is inserted into the groove portion 220 of the tool 200. Thereafter, as indicated by a broken line in FIG. 13, the strike portion 210 strikes a removal action surface 132, which is a surface of the action portion 130 opposite to the action surface 131, while guiding the strike portion 210 by the extension portion 170 and the groove portion 220.

As a result, the action portion 130 is pushed up, a pulling-out force (removal force) from the puncture target site is applied to the needle portion 100 via the second support portion 120 and the first support portion 110, and the needle 101A can be easily removed from the puncture target site.

As a result, the needle 101A can be prevented from being removed from the puncture target site while rotating the needle 101A, and can be removed while using the tool 200. Therefore, it is possible to prevent deformation of the needle 101A, expansion of an inner circumference of the puncture target site, and the like.

In addition, as illustrated in FIGS. 14(a) and 14(b), since the needle 101A has a tapered shape that is gradually tapered from the base end 105a side to the utmost tip end 106a, the needle 101A can easily puncture the puncture target site while maintaining rigidity of the base end 105a (root) side of the needle 101A and suppressing puncture resistance when the needle 101A punctures the puncture target site.

In addition, when the needle 101A is removed from the puncture target site, the needle 101A can be easily removed from the puncture target site while suppressing the bending of the needle 101A or the like.

(Embodiment of Surgical Device of Joint Part)

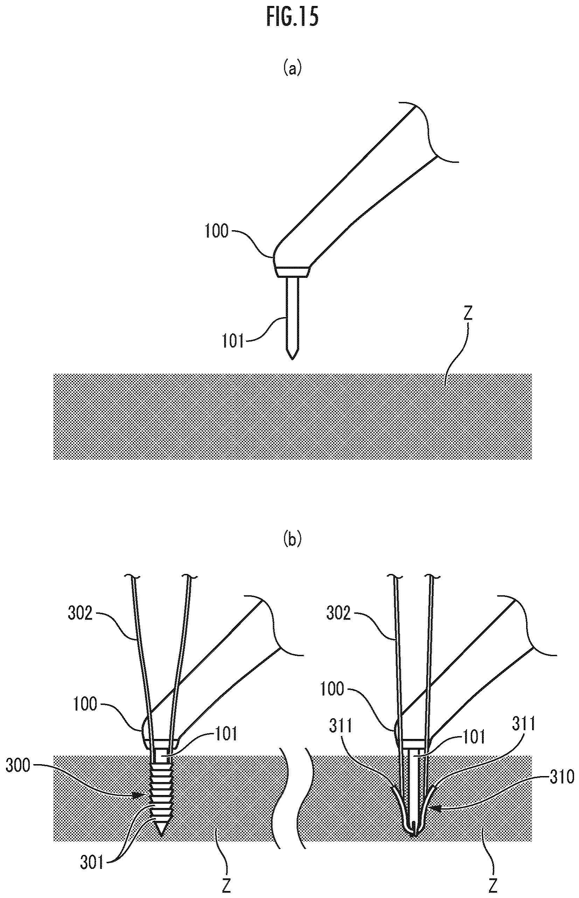

Next, a surgical device of a joint part according to the present invention will be described with reference to FIGS. 15 to 21.

The surgical device of a joint part (hereinafter, also simply referred to as a "surgical device") has a structure in which the needle portion of the microfracture device described above is replaced with various device portions, and the other portions are the same.

That is, the surgical device includes: a device portion pushed into a predetermined site of the joint part to perform a medical treatment on the predetermined site; an action portion 130 having an action surface 131 for applying an external force in a push-in direction of the device portion; a first support portion 110 having a predetermined angle with respect to the push-in direction of the device portion and connected to the device portion; and a second support portion 120 having one end with the action portion 130 and the other end connected to the first support portion 110, in which the device portion, the action portion 130, the first support portion 110, and the second support portion 120 are integrally formed.

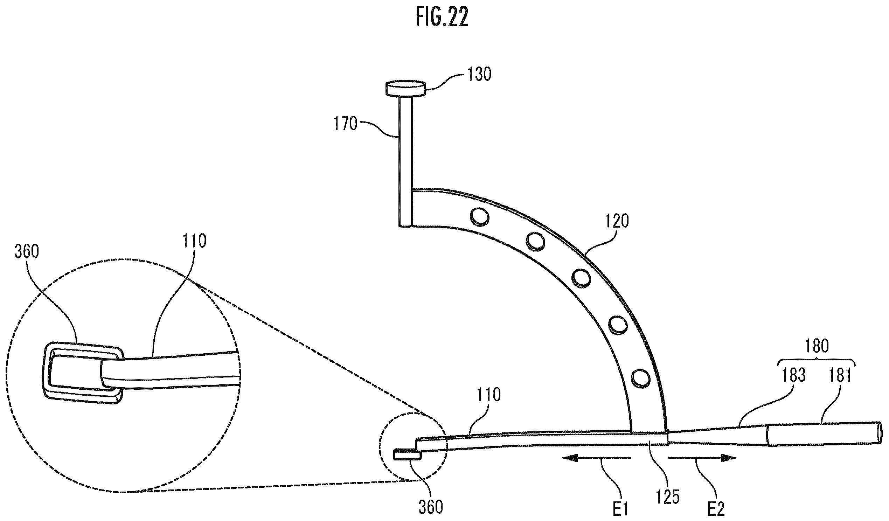

The surgical device illustrated in FIGS. 15 to 21 is different from the surgical device illustrated in FIGS. 22 to 25 described below in that a handle portion does not extend from a connection part between the first support portion 110 and the second support portion 120.

(First Embodiment of Surgical Device of Joint Part)

FIGS. 15 to 17 illustrate a first embodiment of a surgical device of a joint part. In a case of the surgical device, the device portion is an anchor member fixed to a puncture target site Z. In addition, the surgical device can be used for, for example, a repair of medial meniscus posterior root tear, or the like.

An anchor member 300 illustrated on the left side of the paper of FIG. 15(b) is a so-called push-in anchor in which a plurality of annular protrusions 301 having an outer circumferential surface with a tapered shape that gradually decreases in diameter toward a tip end of a needle 101 is consecutively provided in an axial direction, and is detachably mounted on an outer circumference of the needle 101. In addition, a thread 302 having a predetermined length extends from a base end portion side of the anchor member 300.

On the other hand, an anchor member 310 illustrated on the right side of the paper of FIG. 15(b) is a so-called soft anchor that is formed of a fiber tube or the like and has a substantially U-shape with both ends 311 and 311 spaced apart from each other. The anchor member 310 is detachably held at the tip end of a bifurcated needle 101. In addition, the thread 302 having a predetermined length extends from the base end portion side of the anchor member 310.

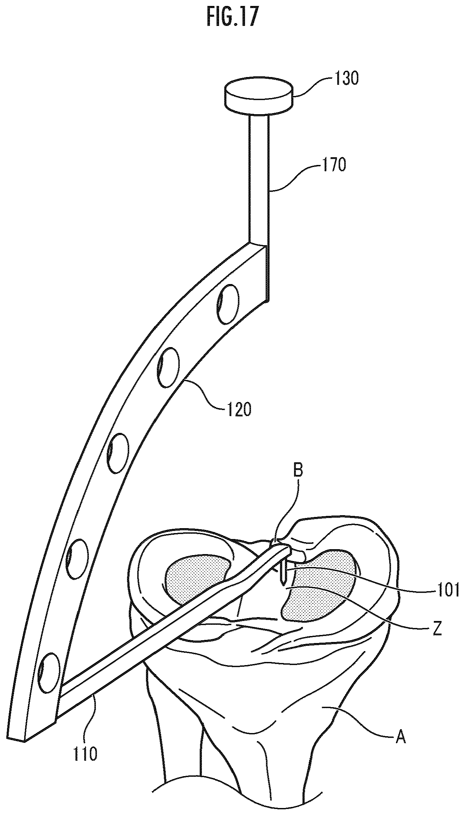

When the surgical device is used, first, as illustrated in FIG. 15(a), the needle 101 punctures the puncture target site Z (here, as illustrated in FIG. 17, refers to a posterior meniscus attachment portion of a tibia A) to form a so-called pilot hole in the puncture target site Z. Thereafter, the anchor member 300 is mounted on an outer circumference of the needle 101, or the anchor member 310 is held by the needle 101, and then the needle 101 is inserted together with the anchor members 300 and 310 into the pilot hole provided in the puncture target site Z.

Then, as illustrated in FIG. 15(b), the annular protrusions 301 of the anchor member 300 or both ends 311 and 311 of the anchor member 310 are caught on an internal tissue of the puncture target site Z, and the anchor members 300 and 310 are fixed to the puncture target site Z. Thereafter, the needle 101 is pulled out from the anchor members 300 and 310, thereby leaving the anchor members 300 and 310 from which the thread 302 has extended.

That is, the anchor members 300 and 310 in a state where the thread 302 extends to the posterior meniscus attachment portion of the tibia A, which is the puncture target site Z, can be fixed.

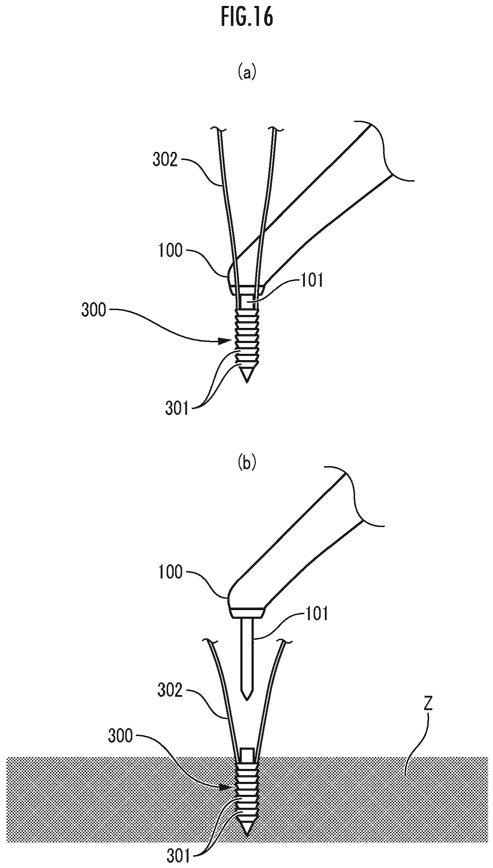

The anchor member may be directly driven into the puncture target site Z without drilling a pilot hole. That is, as illustrated in FIG. 16(a), the anchor member 300 is mounted in advance on the outer circumference of the needle 101. Then, as illustrated in FIG. 16(b), the needle 101 may be punctuated into the puncture target site Z together with the anchor member 300, and the anchor member 300 may be fixed to the puncture target site Z.

Meanwhile, in the conventional repair of medial meniscus posterior root tear, after a bone hole is formed in the posterior meniscus attachment portion of the tibia A, a thread is caught on a meniscal posterior root B (see FIG. 17), and the thread is pulled into the bone hole to repair the torn posterior root of the medial meniscus.

More specifically, a guide pin is inserted into the posterior meniscus attachment portion of the tibia using a guide member, and then a bone hole is formed in the posterior meniscus attachment portion with a drill through the guide pin. Then, after the thread is caught on the meniscal posterior root B, the thread passes through the bone hole, the meniscus is pulled in via the thread, and then the thread is fixed to the tibia A. However, such a procedure is complicated and time-consuming.

On the other hand, in the surgical device of the first embodiment, as described above, the anchor members 300 and 310, which are the device portions, are fixed to the puncture target site Z, and the anchor members 300 and 310 are left on the puncture target site Z, so that the thread 302 can be brought into a state of being pulled out from the posterior meniscus attachment portion of the tibia A.

Therefore, the posterior root tear of the medial meniscus can be repaired only by catching the thread 302 on the meniscal posterior root B and appropriately tying the thread 302. That is, since it is not necessary to perform work such as drilling a bone hole in the posterior meniscus attachment portion with a drill through the guide pin of the guide member as the procedure in the related art, the tear of the posterior meniscus root can be efficiently repaired.

(Second Embodiment of Surgical Device of Joint Part)

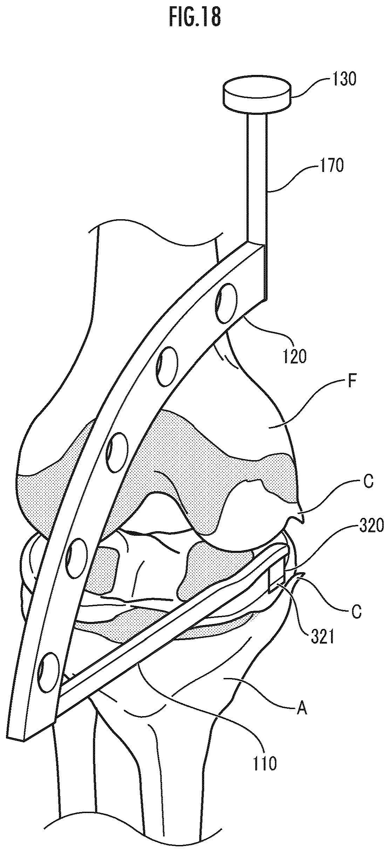

FIG. 18 illustrates a second embodiment of a surgical device of a joint part.

In a case of this surgical device, a curette 320 having a chisel shape is disposed at a tip end of a first support portion 110, and the curette 320 forms a device portion. In addition, the curette 320 has a substantially square plate shape, and a sharp blade 321 is formed at a tip end of the curette 320.

For example, in osteoarthritis of the knee, when a joint space (a gap between the joints) narrows, osteophytes (C) may form at predetermined locations on the tibia A or femur F.

In this case, in the surgical device, since the device portion is a chisel-shaped curette 320 having the blade 321, the chisel-shaped curette 320 is pushed into the osteophyte C, so that the blade 321 of the chisel-shaped curette 320 is easily embedded in the osteophyte C, and the osteophyte C can be quickly removed.

(Third Embodiment of Surgical Device of Joint Part)

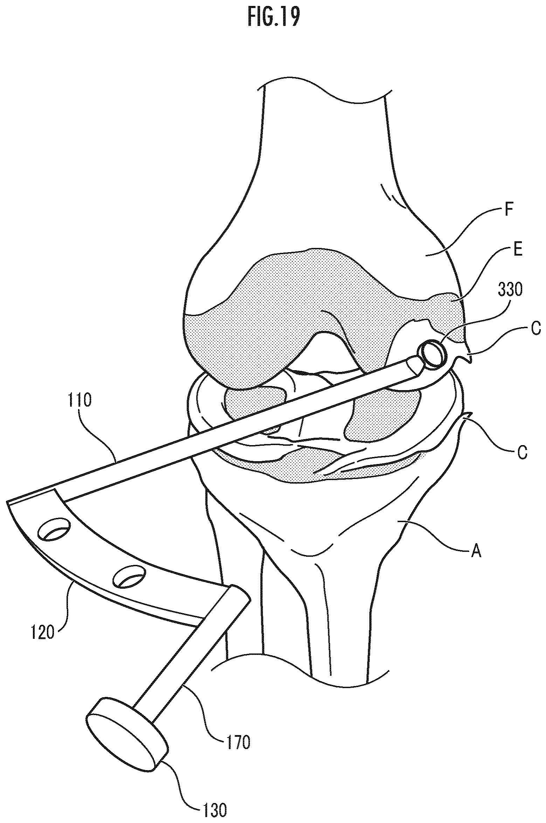

FIG. 19 illustrates a third embodiment of a surgical device of a joint part.

In a case of this surgical device, a curette 330 having a ring shape is disposed at a tip end of a first support portion 110, and the curette 330 forms a device portion. The curette 330 has a substantially annular ring shape.

For example, before performing a medical treatment for repairing cartilage, damaged cartilage or subchondral bone may be removed, but an ossified and hardened tissue E may be present in the vicinity of the damaged cartilage or subchondral bone.

In this case, in the surgical device, since the device portion is the curette 330 having a ring shape, an outer diameter of the surgical device can be formed relatively large, so that the tissue E can be efficiently removed (since the outer diameter of the device is large, the tissue E can be efficiently removed even when the tissue E is formed over a wide range).

(Fourth Embodiment of Surgical Device of Joint Part)

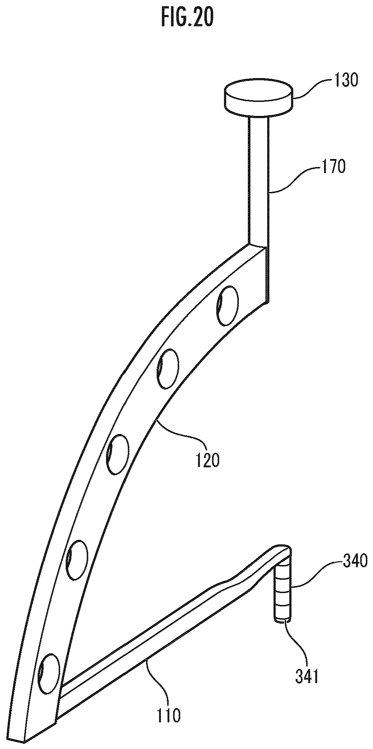

FIG. 20 illustrates a fourth embodiment of a surgical device of a joint part.

In a case of the surgical device, a chisel 340 having a tubular shape is disposed at a tip end of a first support portion 110, and the chisel 340 forms a device portion. The chisel 340 has a substantially cylindrical shape, and a sharp blade 341 having a substantially tapered shape is formed at a tip end of the chisel 340.

For example, there is a mosaicplasty method (autologous osteochondral transplantation method) for treating cartilage injury in the knee joint or the ankle joint. This is a method of collecting a patient's own normal cartilage for each bone and transplanting the normal cartilage to a damaged site of the cartilage in the knee joint or the ankle joint.

In this case, in the surgical device, since the device portion is the chisel 340 having a tubular shape, the chisel 340 can be pushed into the patient's own normal cartilage, and the collected cartilage can be reliably transplanted to the damaged site of the cartilage.

(Fifth Embodiment of Surgical Device of Joint Part)

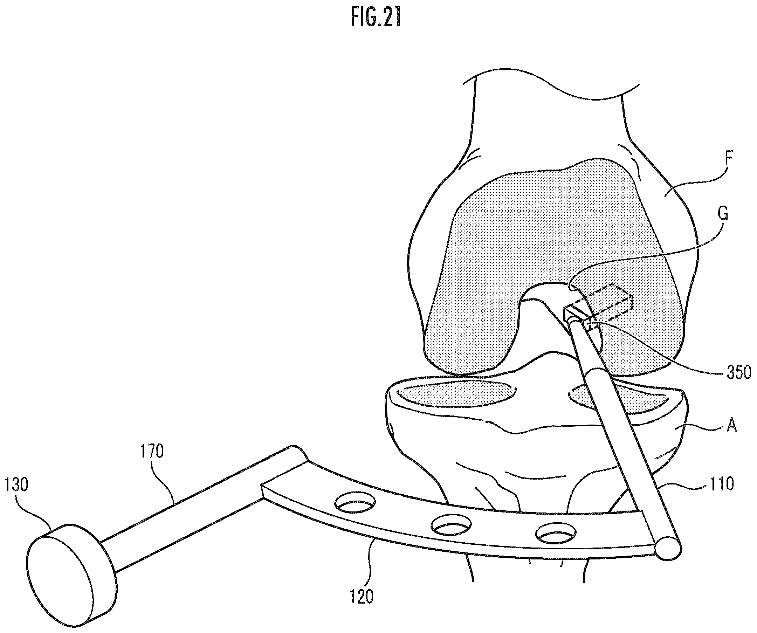

FIG. 21 illustrates a fifth embodiment of a surgical device of a joint part.

In a case of the surgical device, a dilator 350 having a substantially quadrangular block shape is disposed at a tip end of a first support portion 110, and the dilator 350 forms a device portion.

For example, in reconstruction of the anterior cruciate ligament (ACL), a tunnel G is formed in a femur F or a tibia A, and a graft is inserted into the tunnel G and fixed. When the tunnel G is formed, first, a predetermined site such as the femur F is drilled with a drill to have a predetermined size, and then an inner circumferential wall of the hole is compacted to form the tunnel G.

In this case, in the surgical device, since the device portion is the dilator 350, the dilator 350 can be inserted into the inner circumferential wall of the part drilled with the drill, and the inner circumferential wall can be efficiently compacted by striking the inner circumferential wall, so that the tunnel G can be formed with high accuracy.

(Sixth Embodiment of Surgical Device of Joint Part)

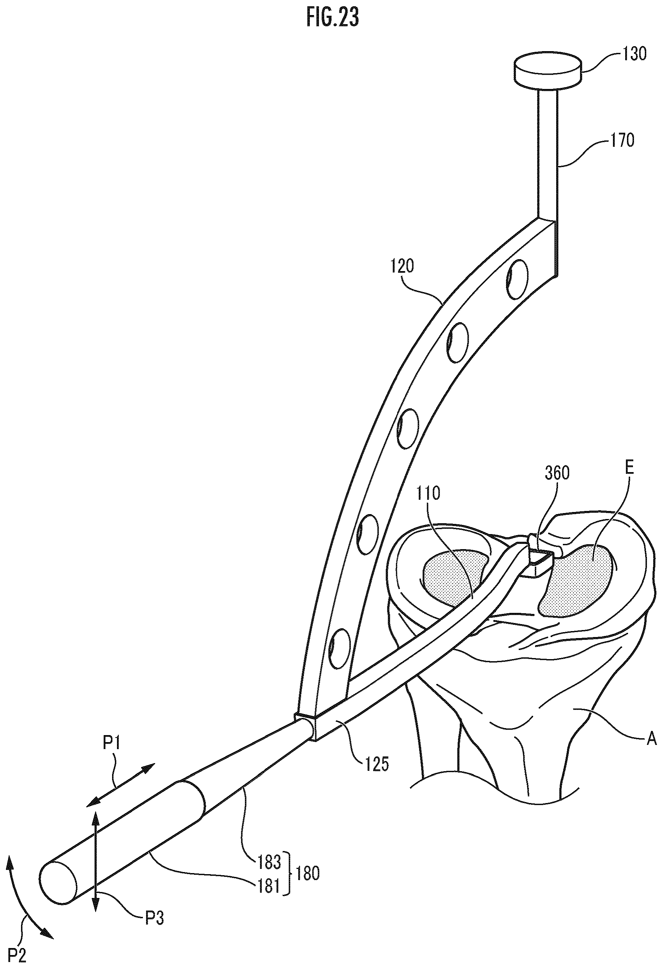

FIGS. 22 and 23 illustrate a sixth embodiment of a surgical device of a joint part. The same reference numerals are assigned to portions that are substantially the same as those in the embodiment, and the description thereof will be omitted.

The surgical device of the sixth embodiment has a structure in which a first support portion 110 extends by a predetermined length, and a handle portion 180, which extends from a connection part 125 between a first support portion 110 and a second support portion 120 in a direction E2 opposite to an extension direction E1 of the first support portion 110 by a predetermined length, is provided.

In addition, the first support portion 110 has a shape in which a tip end part of the first support portion 110 in the extension direction E1 is slightly curved (see FIG. 23), and a curette 360 having a substantially quadrangular annular ring shape is disposed at the tip end part. The curette 360 forms a device portion.

On the other hand, the handle portion 180 includes a base portion 181 having a substantially cylindrical shape and a tip end portion 183 formed by gradually decreasing in diameter from a tip end of the base portion 181 toward an utmost tip end of the handle portion, and an utmost tip end of the tip end portion 183 is connected to the connection part 125 between the first support portion 110 and the second support portion 120.

In addition, as described above, the handle portion 180 is configured to extend from the connection part 125 between the first support portion 110 and the second support portion 120 in the direction E2 opposite to the extension direction E1 of the first support portion 110 by a predetermined length, but the handle portion 180 can also be configured to extend on the same axis as the first support portion 110 from the connection part 125 between the first support portion 110 and the second support portion 120.

In addition, when the surgical device of the sixth embodiment is used, for example, a hardened tissue E generated in a tibia A or the like can be removed using the ring-shaped curette 360, as in the surgical device of the third embodiment illustrated in FIG. 19.

In the surgical device of the sixth embodiment, since a device user can operate the curette 360, which is the device portion, by appropriately operating the handle portion 180 while gripping the handle portion 180 (the device portion can be operated in accordance with a handle portion 180 by operating the handle portion 180), it is possible to support a medical treatment work of a predetermined site using the curette 360, and to improve the simplicity, stability, accuracy, or the like of the medical treatment using the surgical device.

That is, in a state where the device user grips the handle portion 180, for example, (1) as indicated by an arrow P1 in FIG. 23, the handle portion 180 is slid in a front-rear direction so that the curette 360 can be slid in the front-rear direction, (2) as indicated by an arrow P2, the handle portion 180 swings in a left-right direction so that the curette 360 can be swung in the left-right direction, and (3) as indicated by an arrow P3, the handle portion 180 is lifted upward or pushed downward so that the curette 360 can be moved up and down.

Meanwhile, in the surgical device, since the premise is that the surgical device is used for performing a medical treatment while pressing the predetermined site with the device portion by applying an external force (pressing force) to an action portion 130, the device portion is sometimes difficult to operate (since a pressing load is applied to the device portion, the device portion is difficult to operate).

On the other hand, in the embodiment, as described in (1) to (3) above, various operations such as sliding, swinging, and moving up and down of the device portion can be performed via the handle portion 180 by appropriately operating the handle portion 180. As a result, since operability of the device portion can be improved, it is possible to easily perform a medical treatment even at a site where the medical treatment is difficult, and it is possible to improve the stability, accuracy, or the like of the medical treatment.

(Seventh Embodiment of Surgical Device of Joint Part)

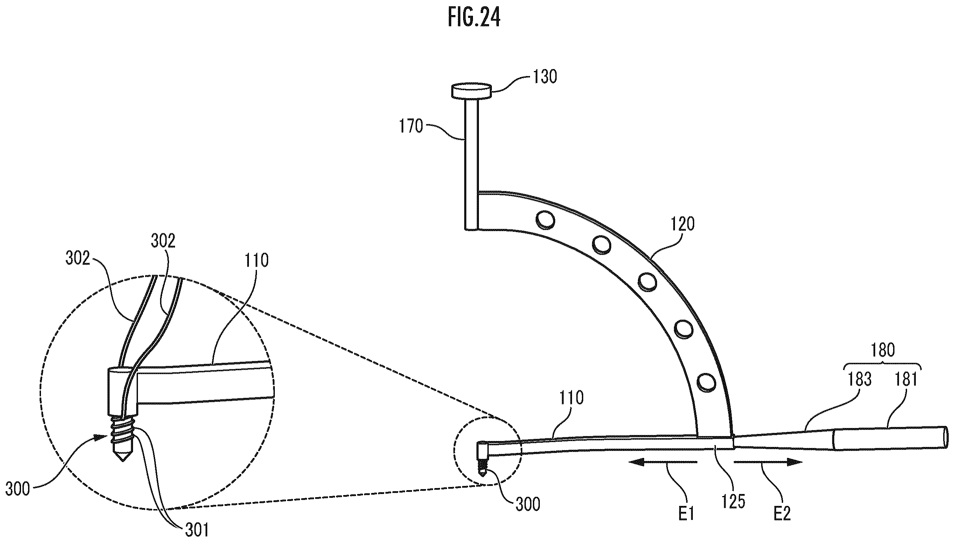

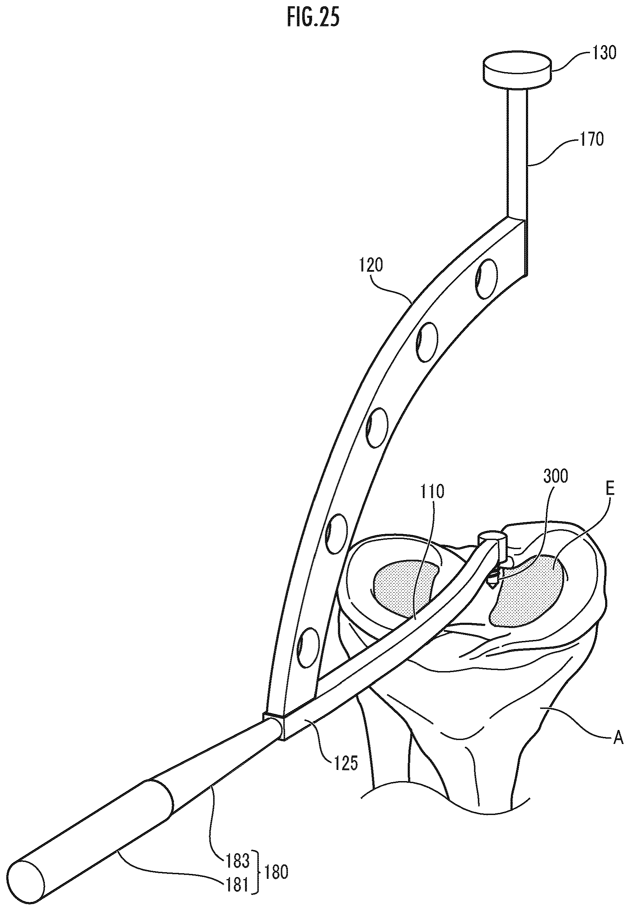

FIGS. 24 and 25 illustrate a seventh embodiment of a surgical device of a joint part. The same reference numerals are assigned to portions that are substantially the same as those in the embodiment, and the description thereof will be omitted.

The surgical device of the seventh embodiment has a structure in which a handle portion 180 is provided, as in the sixth embodiment. In addition, an anchor member 300 as in the surgical device of the first embodiment illustrated in FIGS. 15 to 17 is provided at a tip end of a first support portion 110 that extends by a predetermined length. The anchor member 300 forms a device portion.

Even in the seventh embodiment, as in the sixth embodiment, since the anchor member 300, which is the device portion, can be operated by the device user who grips the handle portion 180 and appropriately operates the handle portion 180, it is possible to support a medical treatment work on the predetermined site using the anchor member 300, and it is possible to improve simplicity, stability, accuracy, or the like of the medical treatment using the surgical device.

The handle portion 180 provided in the sixth and seventh embodiments can also be applied to the second embodiment in which a curette portion 320 having a chisel shape illustrated in FIG. 18 is provided or the fifth embodiment in which the dilator 350 illustrated in FIG. 21 is provided.

Description of Reference Numerals

100, 100': needle portion
101, 101', 101A: needle
110, 110': first support portion
120: second support portion
125: connection part between first support portion and second support portion
130: action portion
131: action surface
132, 150: removal action surface
140: removal action portion
160: grip portion
180: handle portion
200: tool
210: strike portion
220: groove portion
300, 310: anchor member
302: thread
320, 330, 360: curette
340: chisel

350: dilator
D: internal body region
X: cartilage
Y: subchondral bone
Z: puncture target site
A: tibia
B: meniscal posterior root
C: osteophyte
E: ossified and hardened tissue
F: femur
G: tunnel

**Claims**

1. A microfracture device used in a microfracture method, comprising:

   a needle portion for puncturing;
   an action portion having an action surface for applying an external force in a puncture direction of the needle portion;
   a first support portion having a predetermined angle with respect to the puncture direction of the needle portion and connected to the needle portion; and
   a second support portion having one end with the action portion and an other end connected to the first support portion,
   wherein the needle portion, the action portion, the first support portion, and the second support portion are integrally formed.

2. The microfracture device according to Claim 1,
   wherein the action portion is provided on an extension line of the needle portion in the puncture direction via the second support portion.

3. The microfracture device according to Claim 1,
   wherein the second support portion extends from the first support portion in the puncture direction of the needle portion, and an end surface of the second support portion opposite to the first support portion is the action surface.

4. The microfracture device according to Claim 3,
   wherein the first support portion extends beyond the second support portion, and has a grip portion at an end portion of the first support portion opposite to the needle portion.

5. The microfracture device according to any one of Claims 1 to 4,
   wherein the first support portion or the second support portion includes a removal action portion that has a removal action surface for applying a removal force in a direction opposite to the puncture direction of the needle portion.

6. The microfracture device according to Claim 1 or 2,
   wherein an extension portion extends between the second support portion and the action portion, the extension portion being inserted into a groove portion formed in a tool that applies a striking force to the action portion.

7. The microfracture device according to Claim 1 or 2,

   wherein the needle portion has a needle that punctures a puncture target site, and
   the needle has a tapered shape that is gradually tapered from a base end side toward a tip end side.

8. A surgical device of a joint part, comprising:

   a device portion pushed into a predetermined site of the joint part to perform a medical treatment on the predetermined site;
   an action portion having an action surface for applying an external force in a push-in direction of the device portion;
   a first support portion having a predetermined angle with respect to the push-in direction of the device portion and connected to the device portion; and

a second support portion having one end with the action portion and an other end connected to the first support portion,

wherein the device portion, the action portion, the first support portion, and the second support portion are integrally formed.

9. The surgical device according to Claim 8,

wherein the first support portion extends by a predetermined length, and

a handle portion is provided while extending by a predetermined length from a connection part between the first support portion and the second support portion in a direction opposite to an extension direction of the first support portion.

10. The surgical device according to Claim 8,

wherein the first support portion extends by a predetermined length, and

a handle portion does not extend from a connection part between the first support portion and the second support portion.

11. A design method of the microfracture device according to Claim 4, comprising:

a center-of-gravity calculation step of calculating a center of gravity of the microfracture device;

a provisional determination step of provisionally determining the action portion such that the external force applied via the action surface passes through the center of gravity;

a verification step of verifying whether or not the action portion, which has been provisionally determined by the provisional determination step, is located in an internal body region where a medical treatment is expected, when the microfracture method is applied; and

an action position determination step of determining a position where the center of gravity and the action portion are moved to an external body region as an action position of the action portion by adjusting at least a mass of the grip portion, when the action portion is located in the internal body region by the verification step.

12. The design method of the microfracture device according to Claim 11, further comprising:
an action surface shape determination step of determining a shape of the action surface by evaluating a deviation of a traveling direction of the needle portion according to a position of the action surface where the external force is applied.

FIG.1A

## FIG.1B

# FIG.2

θ = 90°          θ = 60°          θ = 45°          θ = 30°

EP 4 663 137 A1

FIG.3

## FIG.4A

# FIG.4B

160

140

150

131 (130)

120

110

101

FIG.5

$\theta = 90°$

$\theta = 60°$

# FIG.6

θ = 45°

θ = 30°

FIG.7

EP 4 663 137 A1

FIG.8

## FIG.9

REAR END

FRONT END

131

110

5mm

STRIKE PORTION PEDESTAL
Φ 5mm

101

DEPTH OF HOLE 7mm

DEVIATION OF HOLE

# FIG.10

## (a) θ: CASE WHERE ANGLE BETWEEN FIRST SUPPORT PORTION AND NEEDLE IS 60°

### DEVIATION WIDTH OF HOLE ACCORDING TO LOCATION AND SIZE OF STRIKE POINT OF ACTION SURFACE

—— DEVIATION WIDTH OF HOLE (mm)   - - - - A (mm)

Z*: HORIZONTAL DISTANCE FROM STRIKE PORTION OF FIRST SUPPORT PORTION TO CENTER OF GRAVITY OF FIRST SUPPORT PORTION + GRIP PORTION

## (b) θ: CASE WHERE ANGLE BETWEEN FIRST SUPPORT PORTION AND NEEDLE IS 30°

### DEVIATION WIDTH OF PERFORATION ACCORDING TO LOCATION AND SIZE OF STRIKE POINT OF ACTION SURFACE

—— DEVIATION WIDTH OF HOLE (mm)   - - - - A (mm)

EP 4 663 137 A1

# FIG.11A

FIG.11B

# FIG.12A

# FIG.12B

## FIG.13

# FIG.14

## (a)

## (b)

# FIG.15

(a)

(b)

# FIG.16

(a)

(b)

# FIG.17

FIG.18

## FIG.19

# FIG.20

FIG.21

# FIG.22

EP 4 663 137 A1

# FIG.23

FIG.24

EP 4 663 137 A1

# FIG.25

## INTERNATIONAL SEARCH REPORT

| | International application No. |
|---|---|
| | **PCT/JP2024/007225** |

---

**A. CLASSIFICATION OF SUBJECT MATTER**

*A61B 17/16*(2006.01)i; *A61B 17/56*(2006.01)i

FI: A61B17/16; A61B17/56

According to International Patent Classification (IPC) or to both national classification and IPC

**B. FIELDS SEARCHED**

Minimum documentation searched (classification system followed by classification symbols)

A61B17/16; A61B17/56

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2016-511099 A (SMITH & NEPHEW, INC.) 14 April 2016 (2016-04-14) paragraph [0033], fig. 10 | 1, 3-4, 6-10 |
| Y | | 5 |
| A | | 11-12 |
| X | WO 2010/053007 A1 (NAKAMURA, Shu) 14 May 2010 (2010-05-14) paragraphs [0002]-[0003], [0011]-[0013], fig. 4-5 | 1-2, 6-10 |
| Y | | 5 |
| Y | WO 2021/229845 A1 (MEDMETALEX CO., LTD.) 18 November 2021 (2021-11-18) paragraph [0028] | 5 |
| A | US 2012/0290020 A1 (MERIDEW, Jason D.) 15 November 2012 (2012-11-15) paragraphs [0015]-[0027], fig. 1-3 | 1-10 |
| A | JP 2002-355254 A (ETHICON, INC.) 10 December 2002 (2002-12-10) paragraph [0040], fig. 33-36 | 1-10 |

☑ Further documents are listed in the continuation of Box C.    ☑ See patent family annex.

| | |
|---|---|
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **15 March 2024** | **02 April 2024** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

**INTERNATIONAL SEARCH REPORT**

| International application No. |
| --- |
| **PCT/JP2024/007225** |

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2009-529987 A (SMITH & NEPHEW, INC.) 27 August 2009 (2009-08-27) paragraph [0026], fig. 8 | 11-12 |

Form PCT/ISA/210 (second sheet) (July 2022)

## INTERNATIONAL SEARCH REPORT
### Information on patent family members

| International application No. |
| --- |
| **PCT/JP2024/007225** |

| Patent document cited in search report | | | Publication date (day/month/year) | Patent family member(s) | | | Publication date (day/month/year) |
| --- | --- | --- | --- | --- | --- | --- | --- |
| JP | 2016-511099 | A | 14 April 2016 | JP | 2019-63582 | A | |
| | | | | US | 2014/0336656 | A1 | |
| | | | | paragraph [0041], fig. 10 | | | |
| | | | | WO | 2014/150193 | A1 | |
| | | | | EP | 2967586 | B1 | |
| | | | | CN | 105025822 | A | |
| WO | 2010/053007 | A1 | 14 May 2010 | US | 2011/0213370 | A1 | |
| | | | | paragraphs [0005]-[0006], [0017]-[0019], fig. 4-5 | | | |
| | | | | JP | 2010-110597 | A | |
| WO | 2021/229845 | A1 | 18 November 2021 | US | 2022/0240948 | A1 | |
| | | | | paragraph [0052] | | | |
| | | | | JP | 2021-178081 | A | |
| | | | | JP | 6781495 | B1 | |
| | | | | EP | 3949878 | A1 | |
| | | | | CN | 113966199 | A | |
| US | 2012/0290020 | A1 | 15 November 2012 | US | 2014/0228850 | A1 | |
| JP | 2002-355254 | A | 10 December 2002 | US | 2001/0027322 | A1 | |
| | | | | paragraph [0078], fig. 33-36 | | | |
| | | | | EP | 1234545 | A2 | |
| JP | 2009-529987 | A | 27 August 2009 | US | 2007/0270870 | A1 | |
| | | | | paragraph [0042], fig. 8 | | | |
| | | | | WO | 2007/106895 | A2 | |
| | | | | EP | 2001374 | A2 | |

Form PCT/ISA/210 (patent family annex) (July 2022)

**REFERENCES CITED IN THE DESCRIPTION**

**Patent documents cited in the description**

- JP 2016511099 W
- US 2021386432
- US 2021177436
- US 2010249786